# EUROPÄISCHE PATENTSCHRIFT

(11) **EP 3 785 659 B1**
(45) Veröffentlichungstag und Bekanntmachung des Hinweises auf die Patenterteilung: **05.07.2023**
(21) Anmeldenummer: 20192791.0
(22) Anmeldetag: 26.08.2020
(51) Int. Cl.: A61B 18/22, A61N 5/06, A61B 18/00

(54) **BELEUCHTUNGSSYSTEM MIT EINEM LICHTLEITER UND EINEM ABSTRAHLELEMENT**
ILLUMINATION SYSTEM WITH A LIGHT CONDUCTOR AND A RADIATING ELEMENT
SYSTÈME D'ÉCLAIRAGE DOTÉ D'UN CONDUCTEUR DE LUMIÈRE ET D'UN ÉLÉMENT RAYONNANT

(30) Priorität: 02.09.2019 DE 102019123448
(43) Veröffentlichungstag der Anmeldung: 03.03.2021
(73) Patentinhaber: SCHOTT AG, 55122 Mainz (DE)
(72) Erfinder: SCHULTHEIS, Bernd, 55270 Schwabenheim (DE); RUSSERT, Hubertus, 55270 Jugenheim (DE); KEIPER, Oliver, 65510 Hünstetten (DE); DIETRICH, Andreas, 55452 Guldental (DE)
(74) Vertreter: Blumbach · Zinngrebe Patentanwälte PartG mbB

(56) Entgegenhaltungen:
- EP-A2- 2 407 807
- DE-B3-102007 028 081
- US-A- 5 290 275
- US-A- 5 754 716

## Beschreibung

Die Erfindung betrifft ein Beleuchtungssystem, umfassend wenigstens eine Laser-Lichtquelle mit einer numerischen Apertur NA_{L}, und einen Lichtleiter, der an einem proximalen Ende an die wenigstens eine Laser-Lichtquelle mittels eines Steckers, welcher ein Steckergehäuse aufweist, anschließbar und/ oder dieser zuordenbar ist, und welches am distalen Ende des Lichtleiters ein Abstrahlelement aufweist.

Derartige Beleuchtungssysteme kommen verstärkt im medizinischen Umfeld zum Einsatz. Derzeit lassen sich folgende Anwendungsschwerpunkte klassifizieren:
- Photodynamische Therapie (PDT) beziehungsweise Photo-Immuno-Therapie (PIT) zur Tumortherapie
- Endovenöse Lasertherapie (EVLT) zur Behandlung von Krampfadern
- Laser induzierte interstitielle Thermotherapie (LITT) sowie
- sonstige Anwendungen, u.a. im Bereich der Dentalmedizin, Augenheilkunde sowie Dermatologie.

Die Photodynamische Therapie (PDT) ist eine minimal-invasive Therapiemöglichkeit bei verschiedenen Krebserkrankungen. Unter der PDT versteht man ein Verfahren zur Behandlung von Tumoren und anderen Gewebeveränderungen (wie beispielsweise Gefäßneubildungen) mit Licht in Kombination mit einer lichtaktivierbaren Substanz. Zu Beginn der Behandlung werden den Patienten intravenös lichtsensible Substanzen, sogenannte Photosensitizer in die Blutbahn injiziert, die sich in beziehungsweise an den Krebszellen anreichern. Diese natürlichen Photosubstanzen konzentrieren sich in den Tumorzellen und bewirken dort eine starke Lichtempfindlichkeit. Dazu werden während der PDT-Behandlung in das Tumor-Gewebe mehrere Kanülen (typisch bis zu 8) gestochen, in die jeweils ein Lichtleiter mit einem DiffusorElement eingeführt wird, wobei die Diffusor-Elemente möglichst räumlich über das Tumor-Gewebe verteilt angeordnet sein müssen. Laser-Licht, in der Regel mit Wellenlängen im sichtbaren Spektralbereich, zum Beispiel Grün-Licht mit 532 nm oder Rot-Licht mit 690 nm Wellenlänge, wird über die Lichtleiter in die Diffusor-Elemente eingekoppelt, so dass das Tumor-Gewebe möglichst gleichmäßig von innen ausgeleuchtet wird. Dabei bilden sich in diesen Zellen aggressive Sauerstoffradikale, welche die Tumorzellen selektiv zerstören. Im Gegensatz zu den kranken Zellen bleiben die gesunden Zellen von dieser chemischen Reaktion unberührt. Der genaue Wirkmechanismus ist unter anderem in "Photodynamic Therapy of Cancer", Cancer Medicine 2003, beschrieben.

Ein ähnliches Verfahren stellt die Photo-Immuno-Therapie (PIT) dar, bei der in Anwesenheit eines photoaktivierbaren Medikaments eine Immunreaktion ausgelöst wird und die Krebszellen dadurch absterben.

Man unterscheidet hier zwischen Zylinder-Diffusoren mit typ. aktiven Längen von 10 bis 50 mm, Spot-Diffusoren oder auch Frontal-Diffusoren, die einen vorwärts gerichteten Beleuchtungskegel erzeugen, sowie Punktstrahler oder sphärische Strahler beziehungsweise Diffusoren, die eine radiale Lichtemission aufweisen.

Bei den Zylinder-Diffusoren kommt es im Betriebszustand insbesondere auf eine möglichst homogene seitliche Abstrahlung der Diffusor-Elemente über deren Länge an. Dies sowohl axial, das heißt an allen Punkten entlang jeder Linie vom proximalen zum distalen Ende in Richtung der Längsachse ist die Abstrahlungsintensität im Rahmen der Homogenitätsanforderung gleich, als auch radial, das heißt an allen Punkten jeder Umfangslinie entlang der Längsachse ist die Abstrahlungsintensität im Rahmen der Homogenitätsanforderung ebenfalls gleich, womit diese Diffusoren nahezu als Lambert'sche Strahler wirken.

Bei allen vorgenannten Diffusoren kann es erwünscht sein, dass die Abstrahlung nicht in alle möglichen Raumrichtungen des jeweiligen Diffusors mit gleicher Intensität erfolgt. In vorteilhafter Ausführung können die Diffusoren entsprechend der geforderten Abstrahlcharakteristik zumindest teil- oder abschnittsweise, beispielsweise raster- oder gradientenartig mit Vorrichtungen versehen sein, die transparent, mit verminderter Transmission, und/oder transluzent und/ oder opak sind, um die Transmission abgestrahlter Strahlung zumindest zu verringern und/oder vollständig zu unterdrücken, versehen sein. Diese können über einen Wellenlängenbereich oder auch wellenlängenselektiv und somit als Filter ausgelegt sein. Ebenso ist denkbar, dass diese eine gerichtete oder diffuse Reflektivität in den jeweiligen Diffusor hinein oder zurück ermöglichen, die beispielsweise als Beschichtungen, Lackierungen oder Bedruckungen oder eine Kombination daraus umfassend ausgeführt sein. Dabei kann eine Beschichtung auch metallische und/oder dielektrische Schichten umfassen.

Gleichzeitig muss auch eine hohe Streueffizienz erzielt werden, um möglichst einen geringen Wärmeeintrag ins Gewebe sicher zu stellen. Typische Homogenitätsanforderungen an die seitliche Abstrahlung liegen bei maximal
± 10 bis 20 % Abweichung von der mittleren Intensität, wobei eine vorwärts gerichtete Abstrahlung, insbesondere aus dem distalen Ende heraus, von mehr als 10% des eingekoppelten Lichtes, typischerweise max. 5 %, zu vermeiden ist. Die typische Laser-Leistung liegt bei den POT-Anwendungen bei < 5 W Dauerleistung, so dass pro cm Diffusorlänge max. zwischen 100 mW und
1000 mW, typischerweise zwischen 200 mW und 500 mW, abgestrahlt werden. Dies erlaubt derzeit den Einsatz von Kunststoff-basierten Diffusor-Ansätzen.

Bei der EVLT führt der behandelnde Arzt über eine winzige Punktionsstelle einen Katheder in die betroffene Vene ein, der als Leitschiene für den Venenlaser dient. Durch gezielte seitliche Abstrahlung der Laserenergie mittels des Diffusors wird die Gefäßinnenwand anschließend stark erwärmt, wodurch die Vene kollabiert und verschlossen wird. Der krankhafte Rückfluss des venösen Blutes wird somit unterbunden. In der Folge verhärtet die Vene, bildet sich zurück und kann vom Körper abgebaut werden. Dabei werden derzeit in der Regel sogenannte Ring- oder Doppelring-Fire-Systeme als Abstrahlelement verwendet. Das Laserlicht wird in Form eines relativ scharf begrenzten Ring- oder Doppelringlichtes radial an das die Vene umschließenden Gewebes abgegeben. Dabei wird der Lichtleiter mit dem Abstrahlelement zur gleichmäßigen Behandlung oft manuell mit möglichst konstanter Geschwindigkeit durch den zu behandelnden Venenabschnitt gezogen, was die Applikation erschwert, da bei Nichtbeachtung beziehungsweise zu langer Verweildauer an einer Stelle weitere Zellschädigungen entstehen können.

Vorteile würde hier ein Zylinder-Diffusor mit sich bringen, wie er bei PDT-Anwendungen zum Einsatz kommt. Allerdings sind bei der EVLT-Behandlung deutlich höhere Laser-Leistungen erforderlich. So beträgt die Laserleistung typisch zwischen 10 und 50 W bei Wellenlängen im NIR-Bereich, das heißt zwischen etwa 800 nm und 1480 nm, welche derzeit mit Dioden-Lasern (zum Beispiel
810 nm, 940 nm oder 1480 nm) oder Nd: YAG-Lasern (1064 nm) bereitgestellt wird. Inzwischen haben sich auch größere Wellenlängen um 2 µm zur EVLT-Behandlung etabliert. Zum Einsatz kommen dann beispielsweise Tm: YAG-Laser (1,9 µm) und Ho: YAG-Laser (2,1 µm). Aufgrund der Absorptionseigenschaften von Gewebe werden bei diesen Wellenlängen geringere Laserleistungen, typischerweise < 10 W, benötigt. Allerdings kommen hier bereits zwingend Quarzglas-Lichtleiter insbesondere für die Zuführung des Laserlichtes zum Einsatz.

Die Homogenitätsanforderungen an die seitliche Abstrahlung von Diffusoren, welche für EVLT eingesetzt werden können, sind gegenüber einer POT-Anwendung weniger hoch und können bei maximal ±30 % bis maximal ± 50 % Abweichung von der mittleren Intensität betragen.

Bei der LITT handelt es sich um ein minimal-invasives Verfahren, das zur lokalen Tumordestruktion eingesetzt wird. Dabei wird unter bildgebender Kontrolle
(zum Beispiel Sonographie/ MRT) der Tumor punktiert, eine (oder mehrere) Laserfaser(n) in den Tumorherd eingebracht und dieser durch thermische Energie verödet. Zum Einsatz kommen hier insbesondere Nd: YAG-Lasern
(1064 nm) sowie Diffusor-Tip-Applikatoren. Die Laserleistung liegt bei etwa 5 bis 8 W (zum Beispiel in "Laserinduzierte Interstitielle Thermotherapie (LITT) bei malignen Tumoren", BÄK und KBV 01/2002).

Die DE 102017122756 A1 beschriebt eine entsprechende Ausführungsform für einen Zylinder-Diffusor, welcher eine im Wesentlichen radiale, homogene Abstrahlcharakteristik aufweist.

Die noch nicht veröffentlichte DE 102018133338 der Anmelderin beschreibt eine Ausführungsform eines sphärischen Diffusors mit weitgehend kugelförmiger Abstrahlcharakteristik.

Bei all diesen Ansätzen hat sich allerdings gezeigt, dass sowohl die Gleichmäßigkeit als auch die Effizienz der Abstrahlung des Diffusors signifikant von der Numerischen Apertur der Einkopplung oder Einstrahlung, das heißt von der Abstrahlcharakteristik der Strahlung der Lichtquelle, insbesondere einer Laser-Lichtquelle, also von der numerischen Apertur des Lasers NA_{L}, in den Lichtleiter und Überführung der geführten Strahlung in den Diffusor, beispielsweise in einer Quarzfaser abhängt. Vergleichsweise große Schwankungen oder Änderungen beziehungsweise Variation der Laser-NA_{L}, teils durch Verwendung unterschiedlicher Laser-Typen (Art des Lasers oder auch gleicher Laser-Art von unterschiedlichen Herstellern) bedingt und/ oder aber auch durch Fertigungsschwankungen bei den Lasern beziehungsweise Laser-Modulen und/ oder durch Fehljustage und/ oder durch thermische Effekte im praktischen Gebrauch hervorgerufen, führen zu einer deutlichen Schwankungsbandbreite in der Abstrahlcharakteristik und in der Effizienz des Diffusors. Mit anderen Worten, eine geforderte oder spezifizierte Homogenität der Abstrahlungsintensität eines Abstrahlelementes, beispielsweise in Einheiten einer relativen Leuchtdichte beziehungsweise deren Abweichung von einem Mittel- oder Maximalwert, wird bei Änderungen der numerischen Apertur des Lasers NA_{L} nicht erreicht beziehungsweise kann in einem, an sich zunächst abgestimmten, System aus Laser, Lichtleiter und Abstrahlelement bei Änderungen der Laserlichtquelle insbesondere deren NA_{L} nicht erhalten werden oder bleibt nicht erhalten.

Sogenannte Modenmischer können hier die Schwankungsbandbreite reduzieren helfen.

Grundsätzlich kann eine Verwendung von beispielsweise Quarzfasern mit unrundem Kernbereich, beispielsweise sogenannte Hex-Fasern mit 6-eckigem hexagonalen Faserkern, als Moden-Mischer verwendet werden. Allerdings sind diese vergleichsweise teuer in der Herstellung, da hierzu spezielle Pre-Formen benötigt werden.

Aus der Literatur sind auch rein mechanische Verfahren zur ModenMischung bekannt, bei denen die Faser gezielt gebogen wird. So beschreibt beispielsweise die DE 102007028081 B3 eine Lasereinrichtung zur Generierung von Laserstrahlen mit einem bestimmten Strahlprofil mit einem Laser und einem an den Laser gekoppelten Lichtleiter. Dabei ist vorgesehen, dass an der einen Seite des Lichtwellenleiters wenigstens zwei erste Anlageelemente für den Lichtwellenleiter beabstandet zueinander angeordnet sind, sich an der gegenüber liegenden Seite des Lichtwellenleiters zwischen zwei den ersten Anlageelementen ein zweites Anlageelement befindet, und wenigstens eines der Anlagenelemente mit einem Stellmechanismus gekoppelt ist und der Stellmechanismus einen Antrieb für ein Stellelement besitzt, wobei das Stellelement mit einer Wegmesseinrichtung mit elektrischem Messsignalausgang und der Antrieb für das Stellelement, die Wegmesseinrichtung und eine Steuereinrichtung so zusammengeschaltet sind, dass über eine durch die Position der Anlegeelemente bestimmte Krümmung des Lichtwellenleiters ein Mischmode, ein Ring-Mode und ein Ring-Mode mit Kern kontinuierlich nacheinander erzeugbar sind. Diese Schrift beschreibt das Grundprinzip einer mechanischen basierten Modenmischung inklusiver einer Vorrichtung, mit der eine Modenmischung gezielt einstellbar ist. Allerdings werden keine konkreten Ansätze offenbart, wie dies für die eingangs erwähnten Anwendungen kostengünstig und praxistauglich realisiert werden kann.

Die EP 2407807 A2 beschreibt eine Lichtfaseranordnung zum Transport eines für die Materialbearbeitung verwendeten und in einer Grundmode-Strahlqualität am Ausgang einer Ausgangsfaser vorliegenden Laserstrahls mit einer Multimoden-Mischfaser, die zum Mischen der sich in ihr ausbreitenden Moden zumindest auf einem Teil ihrer Länge einen gekrümmten Verlauf aufweist, und deren numerische Apertur NA größer ist als die NA der den Laserstrahl am Eingang der Multimode-Mischfaser bereitstellenden und an den Eingang der Multimode-Mischfaser gekoppelten Ausgangsfaser, und bei der zum Transport des am Ausgang der Multimode-Mischfaser anstehenden Laserstrahls zu einem Prozessort eine Multimode-Transportfaser vorgesehen ist, deren NA gleich oder größer ist als die numerische Apertur der Multimode-Mischfaser. Ein derartiger Ansatz kann zwar für Laser-Bearbeitungsmaschinen zweckmäßig sein, kann aber nicht für zum Beispiel zur medizintechnischen Therapie, insbesondere solcher, bei der Lichtleiter oder einen Lichtleiter umfassende Komponente als sogenannte Disposables, also einmal verwendbare und vorzugsweise miniaturisierte Lichtleitsysteme mit entsprechenden Abstrahlelementen, zum Einsatz kommen.

Ein weiterer Ansatz im Stand der Technik ist beispielsweise, dass mittels 3 kleiner Kugeln in einer speziellen Hülse eine als Lichtleiter dienende Quarzfaser lokal einer punktförmigen mechanischen Belastung ausgesetzt ist. Diese Kügelchen drücken sich dabei in das üblicherweise thermoplastische Buffer-Material der Quarzfaser. Nachteilig ist hierbei, dass je nach Umgebungstemperatur beziehungsweise Einsatztemperatur der so erzeugte mechanische Druck auf die Faser aufgrund einer gewissen plastischen Verformung des Buffer-Materials nicht definiert ist und sich mit der Zeit ändern kann und/ oder sich unkontrolliert, lokale Belastungen oder zu geringe Biegeradien einstellen, die die Faser zumindest schädigen. Zudem stellt ein derartiger Ansatz einen zusätzlichen Aufwand in der Fertigung dar.

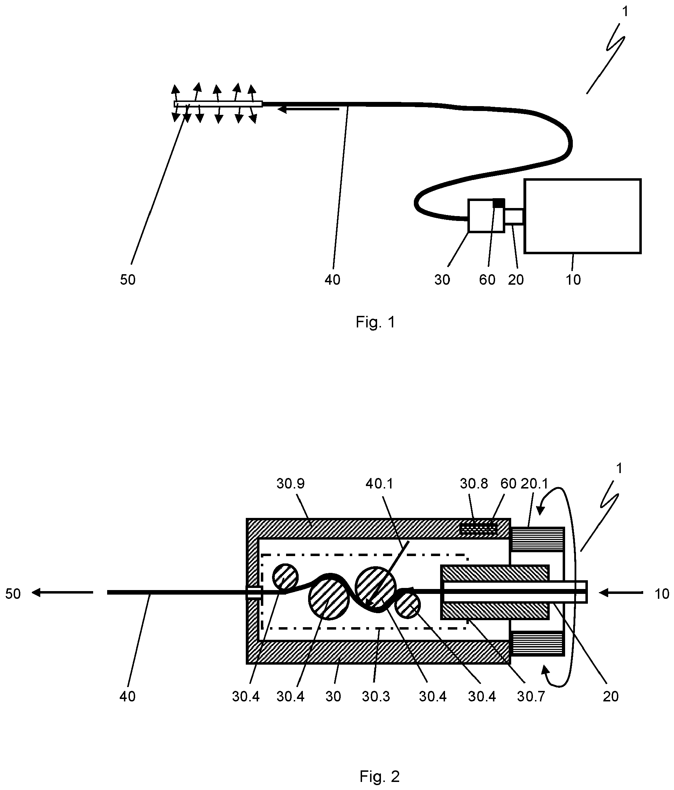

Die US 2018/0113246 A1 beschreibt diverse Ansätze für Zylinder- als auch Frontal-Diffusoren für eine Photo-Immuno-Therapie (PIT), in der unter anderem ebenfalls auf Modenmischer verwiesen wird und beschreibt schematisch in Fig. 2 sowie in den Fig. 39 a bis d verschiedene Ansätze, bei denen die als Lichtleiter verwendete Quarzfaser in Schleifen und dergleichen verlegt wird. Allerdings werden hier keine konkreten Ansätze beschrieben, wie dies hinsichtlich einer gewissen Robustheit einerseits und Praktikabilität andererseits umgesetzt werden kann.

Die US 5 290 275 beschreibt einen Laserkatheter welcher am distalen Ende eine optische Abschirmung und am proximalen Ende einen faseroptischen Koppler aufweist. Die optische Abschirmung kann als ein transparentes Gehäuse aus Quarzglas, Glas oder Saphir oder einem anderen optisch transparenten

Material, das Hitze, Dampf und hoher Laserleistung standhält ausgebildet sein. In einer bevorzugten Ausführungsform wird ein einstellbarer Modenmischer verwendet, um die Moden in den optischen Fasern zu mischen und die Winkeldivergenz von austretenden Laserstrahlen zu erhöhen.

Aufgabe der Erfindung ist es daher, eine kosteneffiziente, insbesondere miniaturisierbare und zur Einwegverwendung geeignete, Lösung für derartige Modenmischer bereitzustellen, die die zuvor genannten Nachteile nicht aufweisen und vor allem eine definierte mechanische Belastung auf den Lichtleiter ausüben, und wobei ein Abstrahlverhalten der Abstrahlelemente, wie sie eingangs erwähnt sind, im Wesentlichen unabhängig von einer Schwankung der numerischen Apertur einer Laser-Lichtquelle ist.

### Offenbarung der Erfindung:

Die Aufgabe der Erfindung wird dadurch gelöst, dass das Steckergehäuse Einrichtungen zur Reduzierung eines Einflusses einer Schwankungsbandbreite der numerischen Apertur NA_{L} der Laser-Lichtquelle des in den Stecker eingekoppelten Laser-Lichtes der Laser-Lichtquelle aufweist, derart, dass das Abstrahlverhalten des Abstrahlelementes im Wesentlichen unabhängig von der Schwankungsbandbreite der numerischen Apertur NA_{L} ist. Damit kann auf eine sehr kostengünstige Art und Weise der Einfluß einer NA_{L}-Schwankung oder Veränderung auf die Abstrahlcharakteristik von zum Beispiel solchen Bauelementen (Abstrahlelementen), wie sie eingangs beschrieben wurden dahingehend reduziert werden, dass eine Schwankungsbandbreite der Einkoppel-NA der Laser-Lichtquelle NA_{L} beziehungsweise einer Einkoppel-Faser nahezu keinen Einfluß mehr auf zum Beispiel die Homogenität der Abstrahlung aber auch auf die Effizienz ausübt. Insbesondere bei den Zylinder-Diffusoren, bei denen es auf einen möglichst gleichmäßigen Intensitätsverlauf über die Diffusor-Länge ankommt, kann dieser mit der erfinderischen Maßnahme deutlich hinsichtlich etwaiger NA-Schwankungen beim Einkoppeln stabilisiert werden. Sind die verschiedenen NAn im System Laser, Lichtleiter, Diffusor nicht abgestimmt, können geforderte, spezifizierte Abstrahlcharakteristika und/ oder Abstrahlintensitäten eines Diffusors und deren räumliche Verteilung nicht erreicht werden. Durch den erfindungsgemäßen Ansatz kann insbesondere in dem Fall, dass sich die NA_{L} der Strahlung des eingesetzten Lasers verändert, sei es nun aufgrund von Veränderungen eines Lasers, beispielsweise durch Alterung, Dejustage, Temperatureinfluß usw., an sich oder aufgrund von Austausch, Ersatz oder Einsatz eines alternativen Lasers, dieser Einfluss auf die Abstrahlcharakteristik beziehungsweise Effizienz eines Abstrahlelementes deutlich verringert werden, und dies mit nahezu keinem Mehraufwand. Dabei ist weiter davon auszugehen, dass in der Regel die NA des Lichtleiters (Zuleitungsfaser), sowie auch des Diffusors (Abstrahlelement) im Wesentlichen konstant bleibt beziehungsweise ist, und diese zueinander passend ausgelegt sind. Letzteres insbesondere auch mit Blick darauf, dass es in kostensensiblen Applikationen nicht vertretbar sein kann und schon gar nicht praktikabel ist, um zum Beispiel auf die Alterung eines Lasers zu reagieren oder Produktionsschwankungen bei an sich gleichen Laser-Modulen auszugleichen, verschiedenste Kombinationen von Zuleitungsfasern und Diffusoren anzubieten oder vorzuhalten. Dementsprechend umfasst das Steckergehäuse zumindest eine Vorrichtung beziehungsweise ist eine Vorrichtung, um den Einfluss einer sich verändernden oder veränderlichen numerischen Apertur eines Lasers auf die Abstrahlung Im Sinne beispielsweise der Abstrahlintensität und -homogenitiät eines Abstrahlelementes zumindest zu reduzieren.

Dabei weist das Steckergehäuse in einem Aufnahmeabschnitt für den Lichtleiter zumindest ein Führungselement zur zumindest teil- oder abschnittsweisen Biegung zumindest einer Stelle des Lichtleiters auf. Durch diese Verbiegung kann erreicht werden, dass sich verschiedene Moden der Lichtausbreitung im Lichtleiter mischen, so dass damit eine weitgehend von NA-Schwankungen der Lichtquelle stabile Abstrahlcharakteristik des Abstrahlelementes erzielt werden kann.

Ein derartiges Führungselement oder auch mehrere Führungselemente, so dass mehrere teil oder abschnittsweise Biegungen des Lichtleiters erreicht werden können, können dabei beispielsweise als zylindrischer und/ oder kegelförmiger, runder Bolzen und/ oder Bolzen mit ovalen oder exzentrischen Querschnittes beziehungsweise Geometrie und/ oder als Kugeln ausgeführt sein, um den Lichtleiter zumindest im Aufnahmeabschnitt räumlich hinsichtlich seiner Lage definiert zu führen und dabei einen bestimmten minimalen Biegeradius, der sich aus der Geometrie und dem Aufbau des Lichtleiters ergibt, nicht unterschreitet.

Mit anderen Worten ist das mindestens eine Führungselement beispielsweise als zylindrischer und/ oder kegelförmiger Bolzen und/ oder als Kugel ausgeführt, um den Lichtleiter zumindest im Aufnahmeabschnitt räumlich hinsichtlich seiner Lage definiert zu führen und dabei zumindest einen Mindest-Biegeradius einzuhalten.

Mit deren spezifischer Ausführungsform, deren Anordnung und/ oder Anzahl und/ oder einheitlicher beziehungsweise kombinierter geometrischer Ausführung kann mittels der Führungselemente die zumindest eine Durchbiegung des Lichtleiters definiert werden.

In einer speziellen Ausführungsvariante kann das Steckergehäuse aus zumindest zwei innen im Steckergehäuse angeordneten Aufnahmeschalen bestehen, wobei zumindest eine der Aufnahmeschale des Gehäuses den Aufnahmeabschnitt zur Aufnahme des Lichtleiters aufweist. Erfindungsgemäß weist der Lichtleiter im Bereich des Aufnahmeabschnitts einen zumindest abschnittsweise einfach oder mehrfach gebogenen, beispielsweise S-förmigen oder wellenartigen Verlauf auf. Durch diesen gebogenen Verlauf kann eine oder mehrere definierte Durchbiegungen des Lichtleiters mit einem oder mehreren bestimmten Biegeradien erzielt werden. Denkbar sind auch andere Verläufe des Lichtleiters, die eine definierte Biegung ergeben. So kann zum Beispiel in einem abgewinkelten Steckergehäuse, beispielsweise als 90°- oder 120°-Ausführung ausgeführt, der Lichtleiter lediglich dieser Biegung folgen, womit bereits eine Modenmischung erzielt werden kann. Allerdings haben sich für die eingangs erwähnten Anwendungen eher gerade ausgeführte Stecker beziehungsweise Steckergehäuse etabliert, so dass hier die zumindest abschnittsweise S-förmige Lichtleiterführung eine bevorzugte Variante darstellt. Denkbar sind auch Anordnungen, bei denen der Lichtleiter in ein oder mehreren Schleifen beziehungsweise sogenannten Loops geführt ist.

Besonders vorteilhaft ist dabei, wenn der Bereich des Aufnahmeabschnitts als Lichtleiter-Aufnahmenut ausgeführt ist. Darin kann der Lichtleiter in seiner Lage definiert mit den gewünschten Biegeradien geführt werden. Die Lichtleiter-Aufnahmenut ist in zumindest einer Aufnahmeschale, bevorzugt U-förmig, ausgebildet. Hinsichtlich der Geometrie beziehungsweise Abmessungen der Lichtleiter-Aufnahmenut hat sich herausgestellt, dass die Tiefe der Lichtleiter-Aufnahmenut mindestens 1,1-mal, vorzugsweise mindestens 2-mal dem Durchmesser des Lichtleiters entsprechen sollte. Damit kann beim Zusammenbau erreicht werden, dass der Lichtleiter zumindest während des Zusammenbaus gehalten beziehungsweise fixiert werden kann.

Erleichtert werden kann die Montage, wenn direkt an der Lichtleiter-Aufnahmenut zumindest abschnittsweise die Wand der Lichtleiter-Aufnahmenut insbesondere ohne Absatz in ein oder mehrere Fixier-Pins übergehen, welche im zusammengesetzten Zustand des Steckergehäuses, in zur Kontur der Fixier-Pins korrespondierende Aufnahmen, in die jeweils andere Aufnahmeschale greifen. Die Fixier-Pins verhindern hier zusätzlich das Herausspringen der Faser beim Einlegen in die Lichtleiter-Aufnahmenut. Zudem wird damit eine hinreichend genaue Positionierung der Aufnahmeschalen beim Zusammenbau sichergestellt.

Besonders kostengünstig hinsichtlich des Montageaufwandes ist es, wenn das Steckergehäuse mittels Rastverbindungen aus den Einzelelementen zusammensteckbar ist.

Hinsichtlich einer einfachen Handhabung in der Praxis ist es besonders vorteilhaft, wenn das Steckergehäuse einen inneren Bereich mit dem Aufnahmeabschnitt für den Lichtleiter, den Aufnahmeschalen und/ oder den Führungselementen sowie ein Außengehäuse aufweist, wobei der Innere Bereich gegenüber dem Außengehäuse frei drehbar, um mehr als 360° Drehwinkel, ausgeführt ist. Beim Befestigen des Lichtleiters an der Laser-Lichtquelle werden damit Torsionsspannungen vermieden, die beim Lichtleiter Schädigungen hervorrufen können.

Weist das Steckergehäuse zur Identifikation und/ oder zur Speicherung von charakteristischen physikalischen Eigenschaften des Abstrahlelementes mindestens einen RFID-Chip und die Laser-Lichtquelle entsprechende dazu korrespondierende Empfangs- beziehungsweise Leseeinheiten auf, wie dies eine besonders bevorzugte Ausführungsvariante vorsieht, können Daten, zum Beispiel bestimmte Kenngrößen zur Abstrahlcharakteristik des Abstrahlelementes oder zur Effizienz gespeichert werden und beim Betrieb mit der Laser-Lichtquelle beispielsweise eines medizinischen Diagnose- oder Therapiegerätes zur exakten Leistungseinstellung beziehungsweise Kalibrierung der Laser-Lichtquelle ausgelesen und verwendet werden. Zudem können auch Zugangscodes verschlüsselt gespeichert werden, die eine eindeutige Identifikation ermöglichen und damit ggf. nicht-geeignete oder - zugelassene Plagiats-Produkte erkannt werden. Dies erhöht die Sicherheit für den Patienten beziehungsweise des Anwenders. Denkbar ist auch eine Speicherung beziehungsweise Setzen eines sogenannten Flags auf dem RFID-Chip, sobald das Bauteil einmal an der Laser-Lichtquelle angeschlossen war. Falls das Bauteil ein zweites Mal eingesteckt wird, kann durch Auslesen des Flags erkannt werden, dass das Bauteil bereits in Benutzung war und die Laser-Lichtquelle für die Benutzung gesperrt wird. Hiermit kann vermieden werden, dass eigentlich für den einmaligen Gebrauch (Single-Use) vorgesehene Bauteile mehrfach verwendet werden, was insbesondere der Patientensicherheit dienlich ist. Derartige RFID-Chips gibt es in einfachen Ausführungen mit lediglich kleiner Speicherkapazität, beispielsweise zum Speichern einer Kennung, bis hin zu Ausführungsformen, bei denen mehrere kByte an Informationen gespeichert werden können. Das Steckergehäuse weist in bevorzugter Ausführungsform zur Aufnahme eines solchen RFID-Chips einen entsprechenden RFID-Chip-Aufnahmeabschnitt auf.

Denkbar sind auch, ggf. mit eingeschränkter Funktionalität, Ausführungsformen die andere Mechanismen zur Identifikation und/ oder zur Speicherung von Charakteristika und/ oder Sicherheitsdaten, so zum Beispiel Codierung mittels Farbe, veränderlicher Einfärbung, Barcodes und/oder QR Codes.

In einer weiteren vorteilhaften Ausführungsvariante kann vorgesehen sein, dass das Steckergehäuse eine Einrichtung aufweist, bei der der Lichtleiter nach einmaliger Benutzung an der Laser-Lichtquelle beim Entfernen des Steckers aus der Laser-Lichtquelle beziehungsweise beim Lösen der Verbindung zwischen Laser-Lichtquelle und Stecker, beispielsweise mechanisch, zumindest teilweise beschädigt oder gekappt oder verschoben wird. Derartige Vorrichtungen sind aus der US 9,304,271 B2 der Anmelderin bekannt. Darin wird ein Verbindungselement zum einmaligen Verbinden und einmaligen Lösen eines faseroptischen Lichtleiters mit beziehungsweise von einer Lichtquelle beschrieben, umfassend ein Gehäuse mit einer Wandung, welches einen Hohlraum umschließt, einen das Gehäuse und den Hohlraum durchlaufenden faseroptischen Lichtleiter, ein mit einem Verbindungsabschnitt der Lichtquelle korrespondierendes Verbindungsstück zum Herstellen der Verbindung mit der Lichtquelle, wobei der Verbindungsabschnitt nach dem Lösen wiederverwendbar ist, und Mittel zum Verhindern eines nochmaligen funktionsgerechten Gebrauchs des Verbindungselements und/ oder des Lichtleiters. Bei erstmaliger Verwendung des Verbindungselements wird der faseroptische Lichtleiter mittels des Verbindungsstücks mit dem korrespondierenden Verbindungsabschnitt, das sich beispielsweise im Gehäuse der Lichtquelle befindet, verbunden. Anschließend wird die Behandlung, beispielsweise eine PDT-Behandlung, dadurch ausgeführt, dass die von der Lichtquelle erzeugte Strahlung durch den Lichtleiter an die zu behandelnde Stelle des menschlichen Körpers weitergeleitet wird. Nach Beendigung der Behandlung werden der faseroptische Lichtleiter und das Verbindungselement von der Lichtquelle getrennt. Die Mittel sorgen dafür, dass das Verbindungselement und/ oder der Lichtleiter nicht mehr funktionsgerecht verwendet werden können. Ist das Verbindungsmittel nicht mehr funktionstüchtig, so kann keine Verbindung mehr mit der Lichtquelle hergestellt und/ oder keine optimale Lichteinkopplung sichergestellt werden. Letzteres äußert sich in einer deutlichen Abnahme der Effizienz. Wird hingegen der Lichtleiter zerstört oder zumindest teilweise beschädigt, so kann keine Strahlung mehr an die zu behandelnde Stelle des menschlichen Körpers gebracht werden. In diesem Fall ist es zwar denkbar, dass die Verbindung zur Lichtquelle hergestellt werden kann, jedoch können moderne Lichtquellen Kontrolleinrichtungen aufweisen, die dem behandelnden Arzt sofort mitteilen, dass keine oder keine ausreichende Strahlendosis mehr am distalen Ende des Lichtleiters ankommt und ein anderer, unbenutzter Lichtleiter verwendet werden muss. In beiden Fällen wird somit gewährleistet, dass ein bereits verwendeter Lichtleiter nicht ein zweites Mal verwendet wird.

Das Beleuchtungssystem sieht dabei einen Lichtleiter als Multimode-Quarzfaser, wasserarme Quarz-Fasern für Anwendungswellenlängen bis etwa 2,5 µm, bei sehr kurzen Anwendungslängen bis knapp 3 µm, oder besonders wasserangereichte Quarz-Fasern für Anwendungswellenlängen im UV-Bereich, mit runder, im Wesentlichen kreisrunder, hexagonaler oder sonstiger polygonartiger oder unregelmäßig geformter Kern-Querschittsstruktur vor. Durch die zuvor beschriebenen Maßnahmen im Steckergehäuse können insbesondere runde Quarzfasern, welche besonders kostengünstig verfügbar sind, eingesetzt werden. Quarzfasern mit hexagonaler oder sonstiger polygonartiger oder unregelmäßig geformter Kern-Querschittsstruktur helfen zusätzlich, die eingangs beschriebene Empfindlichkeit auf NA-Schwankungen der Laser-Lichtquelle zu verringern, sind aber, wie bereits erwähnt, teurer in der Herstellung. Grundsätzlich können auch andere Fasertypen, wie beispielsweise sogenannte HCS-Fasern, welche einen Quarzkern und ein Polymer-Cladding aufweisen, oder auch IR-Fasern, welche insbesondere IR-Licht im Bereich > 2 µm Wellenlänge übertragen können, zum Einsatz kommen. Beispiele dafür sind u.a. sogenannte Chalcogenid-Fasern, Fluorid-Fasern oder aber auch Hohlkernfasern oder PCF-Fasern. Im sichtbaren Wellenlängenbereich inkl. einem NIR-Bereich bis etwa 1 µm können auch optische Glasfasern (GOF) aus beispielsweise Multikomponentengläsern eingesetzt werden, sowie insofern eine zum Diffusor zu führende Strahlungsmenge dies zulässt auch polymer optische Fasern (POF).

Hinsichtlich eines minimal erlaubten Biegeradius (Mindest-Biegeradius) gibt es in der Fachliteratur Angaben beziehungsweise Richtwerte, dass für eine vorwiegend statische Langzeit-Biegebelastung der Minimal-Biegeradius nicht das 100fache bis 200fache des Durchmessers der Multimode-Quarzfaser bezogen auf deren Mantel-Durchmesser (Kern plus Cladding, ohne Buffer-Material) unterschreiten sollte, was bei einer Quarz-Faser mit den Abmessungen 400/440 µm für den Kern-/Manteldurchmesser (Core/Cladding) 44 mm bis 88 mm minimalem Biegeradius entsprechen würde.

Allerdings beziehen sich diese Angaben auf eine zum Teil unterschiedlich angesetzte Lebensdauer und auf unterschiedliche Bruchausfallwahrscheinlichkeiten, so dass hier kein absolut fester Grenzwert existiert. Zudem kann durch einen sogenannten Prooftest, bei dem die Quarzfaser nach dem Ziehen aus der Preform über eine lineare Anordnung von Rollen gezogen wird, die Quarzfaser hinsichtlich ihrer mechanischen Belastbarkeit bezüglich Biegebelastungen vorselektiert werden. Man spricht hierbei auch von einem Prooftest-Level. Dieser liegt üblicherweise im Bereich von 75 kpsi (entsprechend ca. 517 Mpa) bis 150 kpsi (entsprechend ca. 1034 MPa), je nach verwendeten Rollendurchmesser bei dieser Prüfung. Daher kann der Faktor zwischen minimale Biegeradius und Mantel-Durchmesser auch geringer sein, wenn der Prooftest-Level höher angesetzt ist. In bevorzugter Ausführungsvariante ist daher vorgesehen, dass ein minimaler Biegeradius des als Multimode-Quarzfaser ausgebildeten Lichtleiters im Bereich des Aufnahmeabschnitts mindestens dem 60-fachen, bevorzugt mindestens dem 100-fachen des Durchmessers des Lichtleiters bezogen auf seinen Mantel-Durchmesser entspricht. Ein Biegeradius von ca. 40 mm der Quarzfaser im Bereich des Aufnahmeabschnitts würde dabei bezogen auf die oben genannte Fasergeometrie einem Faktor von ca. 91 entsprechen. Diese Biegung bezogen auf 10 Jahre Lebensdauer könnte demnach mit nahezu 0 % Bruchausfallswahrscheinlichkeit mit einem Prooftest-Level bereits ab 100 kpsi (entsprechend ca. 690 Mpa) aufrechterhalten werden.

In einer weiteren Ausführungsvariante kann dabei vorgesehen sein, dass die als Lichtleiter verwendete Multimode-Quarzfaser im Bereich des Aufnahmeabschnitts, in Richtung der Lichtausbreitung am Ende des Aufnahmeabschnitts nach der Faserbiegung zumindest einen Teilbereich aufweist, bei dem das Cladding beziehungsweise Mantel der Quarzfaser durch ein Beschichtungsmaterial ersetzt ist, welches gegenüber dem Brechungsindex des Kerns der Quarzfaser einen höheren Brechungsindex aufweist. Dies ist vorteilhaft, wenn beispielsweise durch die Biegung der Quarzfaser ein hoher Anteil von Mantelmoden erzeugt werden, welche normalerweise unerwünscht sind. Durch die vorgeschlagene Maßnahme können diese störenden Mantelmoden ausgekoppelt werden.

Dabei wird dieses anteilige Laserlicht, welches ausgekoppelt wird, im Bereich des Aufnahmeabschnitts des Lichtleiters absorbiert. Durch zusätzliche Metall-Teile, im Steckergehäuse, welche als Wärmesenke dienen und insbesondere im Aufnahmeabschnitt angeordnet sind, kann eine bessere Wärmeverteilung erzielt werden, wodurch lokale Überhitzungen vermeiden werden können. Vorteilhaft weist das Beleuchtungssystem in oder am Steckergehäuse, insbesondere im Aufnahmeabschnitt eine Wärmesenke auf.

Besonders vorteilhaft ist es, wenn der Stecker als handelsüblicher SMA-Stecker, zum Beispiel der Typ SMA-905, oder als FC-Stecker ausgebildet ist und das Steckergehäuse einen entsprechenden Stecker-Aufnahmeabschnitt zur verdrehsicheren Aufnahme des Steckers aufweist. Damit kann eine definierte Lage des Lichtleiters im Steckergehäuse und insbesondere im Aufnahmeabschnitt für den Lichtleiter gewährleistet werden. Dabei kann vorgesehen sein, dass eine Überwurfmutter zum Fixieren des Steckers in der Laser-Lichtquelle verdrehsicher in dem Außengehäuse befestigbar ist.

Besonders vorteilhaft im Hinblick auf eine erhöhte Robustheit ist es, wenn der Aufnahmeabschnitt für den Lichtleiter Einrichtungen zum Schutz der Faser, also Schutzelemente, beispielsweise als Knickschutz beziehungsweise zur Zugentlastung ausgeführt, aufweist. Dies kann durch die Lage der Führungselemente als auch durch zusätzliche Klemmstellen im Steckergehäuse erzielt werden. Vorteilhaft ist hier die Verwendung eines Schrumpfschlauches, welcher zumindest abschnittsweise über dem Lichtleiter geschoben ist, und von den Aufnahmeschalen am Lichtleiter fixiert ist.

In besonders bevorzugter Ausführungsform ist das Steckergehäuse mit seinen Einzelkomponenten aus Kunststoff-Spritzgussteilen hergestellt, welche besonders kostengünstig realisiert werden können. Vorteilhaft ist hierbei, dass selbst komplexe Funktionalitäten relativ einfach in das Design einbringbar sind. Im Hinblick auf die Verwendung im medizintechnischen Umfeld ist es weiterhin von Vorteil, wenn der verwendete Kunststoff bevorzugt aus einem biokompatiblen Kunststoff-Material ausgeführt ist, welcher zum Beispiel gemäß den Normen EN ISO 10993-1:2018 beziehungsweise EN ISO 10993-5:2009 beziehungsweise USP Class VI gelistet ist. Zudem sollte das zum Einsatz kommende Material derart ausgewählt sein, dass dieses sterilisierbar, insbesondere Ethylenoxid-sterilisierbar (EO), ist, da diese Sterilisationsmethode insbesondere bei Einweg-Anwendungen (Disposable-Anwendungen oder Single-Use-Anwendungen) im medizintechnischen Bereich von Interesse ist, wie dies die ISO 11135:2014 beschreibt. Hierbei ist insbesondere auf eine Chlor-Freiheit des Materials zu achten, da ansonsten während des EO-Prozesses chlorhaltige Verbindungen entstehen können, die zum einen toxisch sein können und zum anderen nur unvollständig nach dem Sterilisationsprozeß ausgetrieben werden können.

In besonders bevorzugter Ausführungsform weist das Beleuchtungssystem Abstrahlelemente als Zylinder-Diffusor mit im wesentlicher radialer Abstrahlcharakteristik, als sphärischer Diffusor mit im wesentlichen kugelförmigen Abstrahlverhalten oder als ein Frontal-Diffusor mit im wesentlichen homogener Abstrahlcharakteristik in distaler Richtung auf. Zum Einsatz kommen auch Zylinder-Diffusoren mit radialer Abstrahlung, die durch partiell aufgebrachte Schichten, reflektive und/ oder absorbierende Schichten, eine sektional gerichtete Abstrahlcharakteristik, zum Beispiel über nur 90° oder 120° des Umfangs, aufweisen können. Insbesondere bei den Zylinder-Diffusoren, wie sie eingangs erwähnt sind, können mit der erfindungsgemäßen Ausgestaltung des Steckergehäuses definierte Abstrahlcharakteristika, unabhängig von etwaigen NA-Schwankungen, gewährleistet werden, und dies über typische Diffusor-Längen von 40 bis 50 mm. Aber auch bei den Frontal-Diffusoren, welche beispielsweise an deren distalem Ende Linsenelemente aufweisen, beispielsweise sogenannte Grin-Linsen (Gradientenindex-Linsen), läßt sich mit o.g. Maßnahmen die Homogenität beziehungsweise die Strahlqualität verbessern.

In bevorzugter Ausführungsvariante kann erreicht werden, dass bei einer Schwankungsbandbreite der numerischen Apertur NA_{L} der Laser-Lichtquelle des in den Stecker eingekoppelten Laser-Lichtes der Laser-Lichtquelle im Bereich von zumindest 0,08 bis 0,24, bevorzugt auch im Bereich von 0,05 bis 0,30, das Abstrahlverhalten des Abstrahlelementes, welches als Zylinder-Diffusor ausgebildet ist, in der Art schwankt, dass die Abstrahlintensität als relative Leuchtdichte gemessen an der Diffusor-Oberfläche über seine Länge nicht mehr als 40 % bezogen auf deren maximalen Wert der relativen Leuchtdichte, besonders bevorzugt nicht mehr als 20% abfällt. Damit können insbesondere die eingangs erwähnten Probleme minimiert beziehungsweise komplett gelöst werden.

Eine, lediglich erläuternd genannte, Verwendung des Beleuchtungssystems, wie es zuvor in seinen verschiedenen Ausführungsvarianten beschrieben wurde, sieht den Einsatz für eine photodynamische Therapie (PDT) oder Photo-Immuno-Therapie (PIT) beispielsweise zur Tumortherapie, für eine endovenöse Lasertherapie (EVLT) beispielsweise zur Behandlung von Krampfadern, für eine Laser-induzierte interstitielle Thermotherapie (LITT) oder für Anwendungen im Bereich der Dentalmedizin, Augenheilkunde sowie Dermatologie vor, wie diese eingangs beschrieben wurden. Im Bereich Dentalmedizin sind hier insbesondere Applikationen zur Wund- oder Parodontose-Behandlung zu nennen. Darüber hinaus gibt es Anwendungen in der Hirnforschung, bei denen mittels Licht einzelnen Gehirnbereich stimuliert und damit Krankheitssymptome behandelt werden können.

Darüber hinaus sind auch Anwendungen im industriellen Bereich vorstellbar, etwa zur Inspektion von schwer zugänglichen Stellen beispielsweise an oder in einer Maschine, bei denen es insbesondere auf eine homogene Ausleuchtung ankommt, oder auch spektroskopische Anwendungen oder in der Biochemie, bei der durch Licht biochemische In-Vitro-Reaktionen stimuliert werden.

Die Erfindung wird im Folgenden anhand eines in den Figuren dargestellten Ausführungsbeispiels näher erläutert. Es zeigen:
Fig. 1 schematisch ein Beleuchtungssystem mit einem Lichtleiter, einem Abstrahlelement und einem Stecker mit Gehäuse zum Betreiben an einer Laser-Lichtquelle,
Fig. 2 schematisch das Prinzip der erfinderischen Lösung,
Fig. 3 in einer Explosionsdarstellung ein erfindungsgemäßes Steckergehäuse mit den Gehäuse-Einzelteilen und weiteren Komponenten,
Fig. 4 eine Schnittdarstellung des Steckergehäuses,
Fig. 5a und 5b jeweils ein Verlaufsdiagramm, welches den Einfluss auf das Streuverhalten bei einem Zylinderdiffusor auf NA-Schwankungen aufzeigt, und
Fig. 6a und 6b jeweils in weiteren Verlaufsdiagrammen die Auswirkung der erfinderischen Maßnahme auf das Abstrahlverhalten bei einem Zylinder-Diffusor.

Fig. 1 zeigt schematisch den Aufbau eines Beleuchtungssystems 1 gemäß der Erfindung. Beispielhaft ist hier eine medizintechnische PDT-Anwendung dargestellt.

Im gezeigten Beispiel besteht das Beleuchtungssystem 1 aus einer Laser-Lichtquelle 10, welche im Betriebszustand Licht in einem bestimmten Spektralbereich aussendet. Für PDT- oder PIT-Anwendungen, wie sie eingangs beschrieben sind, werden Laser verwendet, die auf den zuvor verabreichten biochemisch modifizierten Farbstoff (Photosensitizer) abgestimmte Wellenlänge üblicherweise im sichtbaren Bereich, beispielsweise im grünen Spektralbereich bei 532 nm oder im roten Spektralbereich bei zum Beispiel 690 nm, aussenden. Ein Lichtleiter 40 ist mit einem Stecker 20 an seinem proximalen Ende an die Laser-Lichtquelle 10 angeschlossen. Am distalen Ende weist der Lichtleiter 40 ein als Zylinder-Diffusor ausgebildetes Abstrahlelement 50 auf. In der Regel werden als Lichtleiter 40 Multimode-Quarz-Fasern verwendet, wobei die Stecker 20 in der Regel als koaxialer Steckverbinder, sogenannte SMA-Stecker oder FC-Stecker, ausgebildet sind, bei denen die Fasern in den Stecker 20 geklebt sind. Vorteilhaft hinsichtlich der thermischen Belastbarkeit können auch Stecker 20 mit Neusilber-Hülsen sein, bei denen der Lichtleiter 40 in die Neusilber-Hülse formschlüssig durch plastische Verformung eingebracht, beispielsweise gecrimpt, ist. Darüber hinaus können bei größeren Laser-Leistungen auch Stecker 20 zum Einsatz kommen, bei denen das Faserende des Lichtleiters 40 durch ein Kegelprisma geschützt ist, was vorteilhaft bei Fehljustagen sein kann.

Des Weiteren weist der Stecker 20 ein Steckergehäuse 30 auf, welches die Handhabung vereinfacht. Diese Steckergehäuse 30 können dabei entsprechend farblich ausgeführt sein beziehungsweise besondere Konturen aufweisen, um eine einfache Identifikation und/ oder Unterscheidung verschiedener Typen und/ oder Einsatzbereiche oder -zwecke zu ermöglichen. Zudem können hier zusätzliche Informationen, wie beispielsweise der Herstellername, Bezeichnungen, Chargen- oder Serien-Nummer (LOT oder S/N), aufgedruckt oder mittels Laser eingraviert sein, welche gemäß den UDI-Vorgaben (Unique Device Indentifier) inzwischen Vorschrift für viele medizintechnische Produkte sind, beispielsweise die entsprechenden FDA-Regularien. In diesem Zusammenhang werden zunehmend auch maschinenlesbare 2D-Pixel-Codes aufgebracht, beispielsweise als sogenannter GS1-Code, die eine Vielzahl von Einzelinformationen enthalten können. Um diese und weitere Informationen zu speichern, welche zum Beispiel für die richtige Einstellung der Laser-Lichtquelle 10 wichtig sind, kann das Gehäuse 30 auch einen sogenannten RFID-Chip 60 aufweisen, der mit entsprechenden Ausleseeinheiten beziehungsweise Empfangseinheiten in der Laser-Lichtquelle 10 kommunizieren kann.

Fig. 2 zeigt in einer weiteren schematischen Darstellung das Prinzip der Erfindung. Dargestellt ist ein Steckergehäuse 30, welches Einrichtungen zur Stabilisierung und Einengung einer Schwankungsbandbreite einer numerischen Apertur NA des in den Stecker 20 eingekoppelten Laser-Lichtes von der der Laser-Lichtquelle 10 kommend aufweist. Erfindungsgemäß besitzt das Steckergehäuse 30 in seinem Inneren in einem Aufnahmeabschnitt 30.3 für den Lichtleiter 40 zumindest ein Führungselement 30.4, vorzugsweise mehrere zur gezielten Biegung des Lichtleiters 40. Das zumindest eine Führungselement 30.4 kann als zylindrischer und/ oder kegelförmiger Bolzen und/ oder als Kugel ausgeführt sein, um die der Lichtleiter 40 zumindest im Aufnahmeabschnitt 30.3 definiert geführt ist, wie dies schematisch die Fig. 2 zeigt. Dabei ist die als Lichtleiter 40 verwendete Quarzfaser in Ihrer Lage räumlich definiert fixiert. Die genaue Geometrie des oder der Führungselemente 30.4 ist dabei derart gewählt, dass der sich daraus ergebende Biegeradius 40.1 der Faser im Bereich um diese Führungselemente 30.4 keinesfalls kleiner als das 60-fache des Faserdurchmessers (hier Mantel- beziehungsweise Cladding-Durchmesser) ist, bevorzugt minimal dem 100-fachen des Faserdurchmessers ist. Am Beispiel einer Quarzfaser mit rundem Kern-Querschnitt und typischen Abmessungen hinsichtlich ihres Aufbaus von Kerndurchmesser/ Cladding-Durchmesser/ Hard-Clad-Durchmesser/ Buffer-Durchmesser von 400/ 440/ 470/ 700 µm ergibt sich ein minimaler Biegeradius 40.1 von 26,4 mm beziehungsweise 44 mm. Im konkreten Ausführungsbeispiel beträgt der minimale Biegeradius 40.1 im Bereich des Aufnahmeabschnitts 30.3 40 mm, was einem Faktor zwischen Biegeradius und Faserdurchmesser, bezogen auf den Durchmesser des Mantels, von etwa 91 entspricht.

Damit ist sichergestellt, dass die Quarzfaser langzeitstabil nicht mechanisch überbelastet ist. Wie die Figur 2 ebenfalls schematisch zeigt, weist das Steckergehäuse 30 zudem einen Stecker-Aufnahmeabschnitt 30.7 zur verdrehsicheren Aufnahme des Steckers 20 auf, welche eine feste Einheit mit dem Aufnahmeabschnitt 30.3 mit den Führungselementen 30.4 bildet. Das Steckergehäuse (30) weist weiterhin ein Außengehäuse 30.9 auf, derart, dass dieses frei drehbar (> 360° Drehwinkel) gegenüber dem inneren Bereich mit dem Aufnahmebereich 30.3 gelagert ist. Zudem kann vorgesehen sein, dass eine Überwurfmutter 20.1 zum Fixieren des Steckers 20 in der Laser-Lichtquelle 10 verdrehsicher in dem Außengehäuse 30.9 befestigbar ist. Denkbar sind hierbei auch Rastverbindungen zur Fixierung des Steckers 20 in der Laser-Lichtquelle 10.

In einer weiteren Ausgestaltungsmöglichkeit kann das Steckergehäuse 30 einen RFID-Chip-Aufnahmebereich 30.8 zur Aufnahme des RFID-Chips 60 aufweisen. Beispielsweise kann dieser RFID-Chip 60 als Platinen-Ring ausgeführt sein.

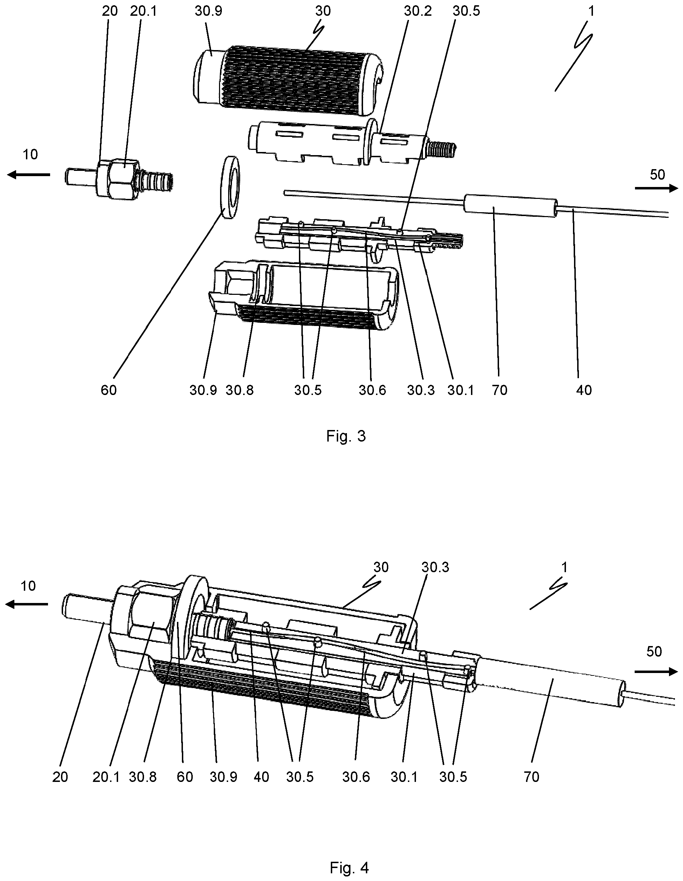

Fig. 3 zeigt schematisch in einer Explosionsdarstellung den Aufbau des Steckergehäuses 30 mit den Gehäuse-Einzelteilen als konkrete Ausführungsform der Erfindung.

Das Steckergehäuse 30 weist demnach zumindest zwei Aufnahmeschalen 30.1, 30.2 auf, bei der zumindest eine der Aufnahmeschalen 30.1 des Gehäuses 30 den Aufnahmeabschnitt 30.3 zur Aufnahme des Lichtleiters 40 ausbildet. Im gezeigten Beispiel weist der Lichtleiter 40 im Bereich des Aufnahmeabschnitts 30.3 einen zumindest abschnittsweise S-förmigen Verlauf auf. Im gezeigten Beispiel ist der Bereich des Aufnahmeabschnitts 30.3 als Lichtleiter-Aufnahmenut 30.6 ausgeführt, welche vorzugsweise U-förmig ausgebildet ist. Dabei ist vorgesehen, dass direkt an der Lichtleiter-Aufnahmenut 30.6 zumindest abschnittsweise die Wand der Lichtleiter-Aufnahmenut 30.6 ohne Absatz in ein oder mehrere Fixier-Pins 30.5 übergehen, welche im zusammengesetzten Zustand des Steckergehäuses 30 in zur Kontur der Fixier-Pins 30.5 korrespondierende Aufnahmen in die jeweils andere Aufnahmeschale 30.2 greifen.

Der Lichtleiter 40 ist dabei im Aufnahmeabschnitt 30.3, hier konkret in der Lichtleiter-Aufnahmenut 30.6 derart geführt, dass der sich damit ergebende Biegeradius den zulässigen Biegeradius nicht überschreitet. Für das gezeigte Beispiel wird als Lichtleiter 40 eine Quarzfaser mit folgenden Geometrien verwendet:

| | |
|---|---|
| - Kerndurchmesser (Core) | 400 µm |
| - Mantel-Durchmesser (Cladding) | 440 µm |
| - Kunststoffmantel-Durchmesser (Hard-Clad) | 470 µm |
| - Äußerer Durchmesser mit Schutzhülle (Buffer) | 700 µm |

Nach den oben aufgezeigten Randbedingungen zu einem minimal zulässigen Biegeradius ergibt sich demnach im gezeigten Beispiel ein Biegeradius von ca. 40 mm.

Die beiden Aufnahmeschalen 30.1, 30.2 bilden im Steckergehäuse 30 einen inneren Bereich, welcher gegenüber dem Außengehäuse 30.9, hier ebenfalls als zwei Halbschalen ausgeführt, um mehr als 360° Drehwinkel frei drehbar gelagert ist. Dabei ist vorgesehen, dass der Stecker 20, im gezeigten Beispiel ein SMA-905-Stecker, fest mit den beiden Aufnahmeschalen 30.1, 30.2 verbunden ist. Das Außengehäuse 30.9 umfasst dagegen eine Überwurfmutter 20.1 des Steckers. Damit kann erreicht werden, dass nach dem Einstecken des Steckers 20 in die Laser-Lichtquelle 10 durch Drehen des Außengehäuses 30.9 die Überwurfmutter 20.1 mitgedreht und damit der Stecker 20 an der Laser-Lichtquelle 10 sicher und definiert hinsichtlich des Abstandes fixiert werden kann.

Im gezeigten Beispiel weist das Steckergehäuse 30 in den beiden Schalen, die das Außengehäuse 30.9 bilden einen RFID-Chip-Aufnahmeabschnitt 30.8 auf, in dem vor dem Zusammenbau noch ein RFID-Chip 60 eingelegt werden kann. Im gezeigten Beispiel ist dieser als ringförmige Platine ausgeführt. Weiterhin ist eine Zugentlastung beziehungsweise ein Knickschutz 70 auf dem Lichtleiter 40 vorgesehen, der als Schlauch, vorzugsweise als Schrumpfschlauch, ausgeführt sein kann. Beim Zusammenbau der beiden Aufnahmeschalen 30.1, 30.2 kann dieser zusätzlich zwischen diesen geklemmt werden. In einer anderen Ausführungsvariante kann der Schrumpfschlauch auch auf die Aufnahmeschalen 30.1, 30.2 aufgeschrumpft sein. Zudem kann diese Zugentlastung beziehungsweise der Knickschutz 70 zusätzlich bedruckt sein, beispielsweise mit Information zum Bauteiltyp, mit einer Seriennummer und/ oder Chargen-Nummer.

Das Steckergehäuse 30 mit seinen zuvor beschriebenen Einzelelementen ist besonders bevorzugt mittels Rastverbindungen zusammensteckbar ausgeführt.

Fig. 4 zeigt in einer 3D-Darstellung das Steckergehäuses 30, wie es in Figur 3 beschrieben wurde, im zusammengebauten Zustand in einer Schnittdarstellung.

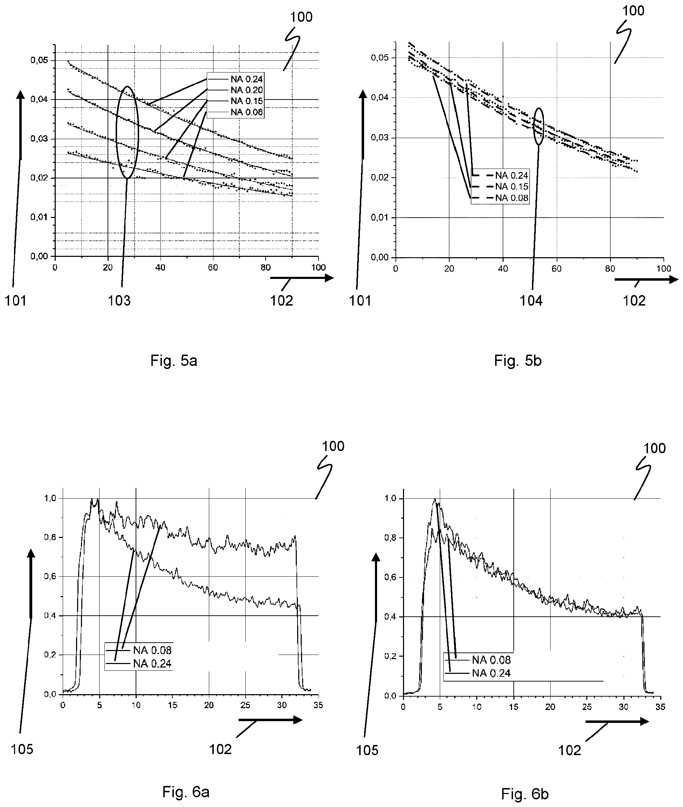

In Fig. 5a und Fig. 5b sind schematisch in jeweils einem Verlaufsdiagramm 100 unterschiedliche Verläufe eines ortsabhängigen Streukoeffizienten 101 in Abhängigkeit vom Abstand zur Lichteinkopplung 102 dargestellt. Der ortsabhängige Streukoeffizient 101 k₍ₓ₎ in mm⁻¹ stellt dabei eine empirisch ermittelte Materialeigenschaft dar, mit welchem Anteil Licht diffus seitlich aus dem Diffusor gestreut wird. Bei einer konstanten Konzentration von Streuelementen entlang der Längsachse eines Diffusor-Grundkörpers weist der Intensitätsverlauf einen typischerweise exponentiellen Abfall mit I₍ₓ₎ = I₀ x e ^{-x/k(x)} auf. Durch eine entsprechende Anordnung und Anzahl der Streuelemente im Diffusor-Grundkörper kann in Verbindung mit der Einkoppel-NA einer Laserlichtquelle der Soll-Verlauf für den ortsabhängigen Streukoeffizienten k₍ₓ₎ gezielt vorgegeben werden.

Fig. 5a zeigt einen gemessenen Kurvenverlauf für Lichteinkopplungen mit unterschiedlicher numerischer Apertur NA in den Stecker 20. Ein Verlaufsbereich ohne Modenmischung 103 zeigt die gemessene beziehungsweise die daraus berechnete Schwankungsbandbreite des ortsabhängigen Streukoeffizienten als Maß für den Anteil des Lichtes, der diffus seitlich aus dem Diffusor gestreut wird und somit auch als Maß für dessen Homogenität, für eine NA von 0,08 bis zu einer NA von 0,24. Dabei zeigt Fig. 5a ohne den Mischer eine starke Abhängigkeit von der Einkoppel-NA am Stecker 20. Zwischen den beiden Extrem-Werten NA = 0,08 und NA = 0,24 ist fast ein Faktor 2 für den Streukoeffizienten festzustellen, was hinsichtlich des Designs in erster Näherung einen Faktor 2 für die Anzahl der Streufilamente entspricht (vergleiche dazu die DE 102017122756 A1).

Bei steckerseitiger Verwendung des Modenmischers ist nur noch eine minimale Abhängigkeit festzustellen, wie dies Figur 5b zeigt. Hier liegt die Kurvenschar mit Modenmischer (Verlaufsbereich mit Modenmischung 104) auf dem Niveau der Messung ohne Mischer bei der maximalen Einkoppel-NA am Stecker 20.

Die Figuren 6a und 6b zeigen in weiteren Verlaufsdiagrammen 100 die Auswirkung des Modenmischers auf einen gemessenen Verlauf für eine relative Leuchtdichte 105 in Abhängigkeit vom Abstand zur Lichteinkopplung 102. Beide Figuren zeigen den typ. Verlauf am Beispiel eines Zylinder-Diffusors mit 40 mm Diffusor-Länge. Der Aufbau de Diffusors entspricht dabei dem Aufbau, wie er in der DE 102017122756 A1 beschrieben ist.

Fig. 6a zeigt dabei die Abhängigkeit von der Einkoppel-NA sehr deutlich. Bei einem auf eine Einkoppel-NA von 0,08 optimiertem Diffusor-Design kann über die Diffusor-Länge eine gute Homogenität mit max. 30% Abfall bezogen auf eine max. Intensität (= 100%) realisiert werden. Wird die Einkoppel-NA beispielsweise auf einen Wert von 0,24 erhöht, beträgt der Abfall des Intensitätsverlaufs bis zu 55% bezogen auf den max. Wert, was in der Regel nicht mehr für PDT- oder PIT-Anwendungen geeignet ist.

Fig. 6b zeigt hingegen die NA-Abhängigkeit der gemessenen relativen Leuchtdichte 105 mit einem Modenmischer, wie er in den Figuren 3 und 4 beschrieben ist. Der Verlauf bei einer Einkoppel-NA von 0,08 und bei 0,24 zeigen sehr ähnliche Verläufe. Natürlich ist hier, wie zuvor beschrieben, der Verlauf an die Max.-NA (hier 0,24) auch bei einer Einkoppel-NA von 0,08 angelehnt. Dies kann aber beim Design des Diffusors durch eine Reduzierung der Anzahl von Streuelementen im Diffusor-Grundkörper gezielt nach korrigiert werden, wie dies in der DE 102017122756 A1 beschrieben ist.

Mit dieser Maßnahme, gekoppelt mit einem erfindungsgemäßen Modenmischer im Steckergehäuse 30, wie er zuvor beschrieben ist, kann erreicht werden, dass bei einem Zylinderdiffusor als Abstrahlelement 50 die Abstrahlintensität als relative Leuchtdichte 105 gemessen an der Diffusor-Oberfläche über seine Länge nicht mehr als 40 % bezogen auf den Max-Wert der relativen Leuchtdichte 105 (= 100 %), besonders bevorzugt nicht mehr als 20% abfällt, wobei die numerischen Apertur NA_{L} der Laser-Lichtquelle 10 und des so in den Lichtleiter 40 im Stecker 20 eingekoppelten Laser-Lichtes der Laser-Lichtquelle 10 in einem Bereich von 0,08 bis 0,24 schwanken kann. In besonders bevorzugter Ausführungsvariante kann der Schwankungsbereich auch größer sein, zum Beispiel zwischen 0,05 und 0,30 sein.

### Bezugszeichenliste:

| | |
|---|---|
| 1 | Beleuchtungssystem |
| 10 | Laser-Lichtquelle |
| 20 | Stecker |
| 20.1 | Überwurfmutter |
| 30 | Steckergehäuse |
| 30.1 | Erste Aufnahmeschale |
| 30.2 | Zweite Aufnahmeschale |
| 30.3 | Aufnahmeabschnitt |
| 30.4 | Führungselement |
| 30.5 | Fixier-Pin |
| 30.6 | Lichtleiter-Aufnahmenut |
| 30.7 | Stecker-Aufnahmeabschnitt |
| 30.8 | RFID-Chip-Aufnahmeabschnitt |
| 30.9 | Außengehäuse |
| 40 | Lichtleiter |
| 40.1 | Biegeradius |
| 50 | Abstrahlelement |
| 60 | RFID-Chip |
| 70 | Schutzelement |
| 100 | Verlaufsdiagramm |
| 101 | Ortsabhängiger Streukoeffizient |
| 102 | Abstand von der Lichteinkopplung am Zylinder-Diffusor |
| 103 | Verlaufsbereich ohne Modenmischung |
| 104 | Verlaufsbereich mit Modenmischung |
| 105 | Relative Leuchtdichte |
| | |
| | |

## Patentansprüche

1. Beleuchtungssystem (1), umfassend wenigstens eine Laser-Lichtquelle (10) mit einer numerischen Apertur NA_{L}, und einen Lichtleiter (40), der an einem proximalen Ende einen Stecker (20) mit einem Steckergehäuse (30) aufweist und der mittels des Steckers (20) an die wenigstens eine Laser-Lichtquelle (10) anschließbar und/oder dieser zuordenbar ist, und welches am distalen Ende des Lichtleiters (30) ein Abstrahlelement (50) aufweist, wobei das Steckergehäuse (30) Einrichtungen zur Reduzierung eines Einflusses einer Schwankungsbandbreite der numerischen Apertur NA_{L} der Laser-Lichtquelle (10) des in den Stecker (20) eingekoppelten Laser-Lichtes der Laser-Lichtquelle (10) aufweist.

2. Beleuchtungssystem (1) nach Anspruch 1, wobei das Steckergehäuse (30) in einem Aufnahmeabschnitt (30.3) für den Lichtleiter (40) zumindest ein Führungselement (30.4) zur zumindest teil- oder abschnittsweisen Biegung des Lichtleiters (40) aufweist.

3. Beleuchtungssystem (1) nach einem der Ansprüche 1 oder 2, wobei das mindestens eine Führungselement (30.4) als zylindrischer und/ oder kegelförmiger Bolzen und/ oder als Kugel ausgeführt ist, wobei der Lichtleiter (40) zumindest im Aufnahmeabschnitt (30.3) räumlich hinsichtlich seiner Lage definiert geführt ist und dabei zumindest ein Mindest-Biegeradius (40.1) eingehalten wird.

4. Beleuchtungssystem (1) nach zumindest einem der Ansprüche 1 bis 3, wobei das Steckergehäuse (30) aus zumindest zwei innen im Steckergehäuse (30) angeordneten Aufnahmeschalen (30.1, 30.2) besteht und zumindest eine der Aufnahmeschalen (30.1,30.2) des Gehäuses (30) den Aufnahmeabschnitt (30.3) zur Aufnahme des Lichtleiters (40) aufweist, wobei der Lichtleiter (40) im Bereich des Aufnahmeabschnitts (30.3) einen zumindest abschnittsweise S-förmigen oder wellenartigen Verlauf aufweist.

5. Beleuchtungssystem (1) nach zumindest einem der Ansprüche 1 bis 4, wobei der Bereich des Aufnahmeabschnitts (30.3) als Lichtleiter-Aufnahmenut (30.6) ausgeführt ist.

6. Beleuchtungssystem (1) nach Anspruch 5, wobei die Lichtleiter-Aufnahmenut (30.6) in zumindest einer der Aufnahmeschalen (30.1, 30.2) eine Tiefe von mindestens 1,1-mal, vorzugsweise mindestens 2-mal dem Gesamtdurchmesser des Lichtleiters (40) aufweist und vorzugsweise U-förmig ausgebildet ist.

7. Beleuchtungssystem (1) nach einem der Ansprüche 5 oder 6, wobei direkt an der Lichtleiter-Aufnahmenut (30.6) zumindest abschnittsweise die Wand der Lichtleiter-Aufnahmenut (30.6) ohne Absatz in ein oder mehrere Fixier-Pins (30.5) übergehen, welche im zusammengesetzten Zustand des Steckergehäuses (30) in zur Kontur der Fixier-Pins (30.5) korrespondierende Aufnahmen in die jeweils andere Aufnahmeschale (30.1, 30.2) greifen.

8. Beleuchtungssystem (1) nach mindestens einem der vorhergehenden Ansprüche mit zumindest einem der folgenden Merkmale
- das Steckergehäuse (30) ist mittels Rastverbindungen aus den Einzelelementen zusammensteckbar,
- das Steckergehäuse (30) weist einen inneren Bereich mit dem Aufnahmeabschnitt (30.3) für den Lichtleiter (40), den Aufnahmeschalen (30.1, 30.2) und/ oder den Führungselementen (30.4) sowie ein Außengehäuse (30.9) auf, wobei der Innere Bereich gegenüber dem Außengehäuse (30.9) um mehr als 360° Drehwinkel frei drehbar ausgeführt ist,
- das Steckergehäuse (30) weist mindestens einen RFID-Chip (60) zur Identifikation und/ oder zur Speicherung von charakteristischen physikalischen Eigenschaften des Abstrahlelementes (40) und die Laser-Lichtquelle (10) entsprechende dazu korrespondierende Empfangsbeziehungsweise Leseeinheiten auf,
- das Steckergehäuse (30) weist eine Einrichtung auf, bei der der Lichtleiter (40) nach einmaliger Benutzung an der Laser-Lichtquelle (10) beim Entfernen des Steckers (20) aus der Laser-Lichtquelle (10) beziehungsweise beim Lösen der Verbindung zwischen Laser-Lichtquelle (10) und Stecker (20) zumindest teilweise beschädigt oder gekappt oder verschoben wird,
- das Steckergehäuse (30) weist eine Wärmesenke, insbesondere im Aufnahmeabschnitt (30.3), auf.

9. Beleuchtungssystem (1) nach mindestens einem der vorhergehenden Ansprüche, wobei der Lichtleiter (40) als Multimode-Quarzfaser mit kreisrunder, hexagonaler oder sonstiger polygonartiger oder unregelmäßig ausgebildeter Kern-Querschnittsstruktur ausgeführt ist.

10. Beleuchtungssystem (1) nach Anspruch 9, wobei ein minimaler Biegeradius (40.1) des als Multimode-Quarzfaser ausgebildeten Lichtleiters (40) im Bereich des Aufnahmeabschnitts (30.3) mindestens dem 60-fachen, bevorzugt mindestens dem 100-fachen des Durchmessers des Lichtleiters (40) bezogen auf seinen Mantel-Durchmesser entspricht.

11. Beleuchtungssystem (1) nach Anspruch 10, wobei die als Lichtleiter (40) verwendete Multimode-Quarzfaser im Bereich des Aufnahmeabschnitts (30.3), in Richtung der Lichtausbreitung am Ende des Aufnahmeabschnitts (30.3) nach der Faserbiegung zumindest einen Teilbereich aufweist, bei dem der Mantel der Quarzfaser durch ein Beschichtungsmaterial ersetzt ist, welches gegenüber dem Brechungsindex des Kerns der Quarzfaser einen höheren Brechungsindex aufweist.

12. Beleuchtungssystem (1) nach mindestens einem der vorhergehenden Ansprüche, wobei der Stecker (20) als SMA- oder FC-Stecker ausgebildet ist und das Steckergehäuse (30) einen entsprechenden Stecker-Aufnahmeabschnitt (30.7) zur verdrehsicheren Aufnahme des Steckers (20) aufweist, wobei eine Überwurfmutter (20.1) zum Fixieren des Steckers (20) in der Laser-Lichtquelle (10) verdrehsicher in dem Außengehäuse (30.9) befestigbar ist.

13. Beleuchtungssystem (1) nach mindestens einem der vorhergehenden Ansprüche, wobei der Aufnahmeabschnitt (30.3) ein mechanisches Schutzelement (70) für den Lichtleiter (40), insbesondere eine Zugentlastung und/ oder einen Knickschutz aufweist.

14. Beleuchtungssystem (1) nach mindestens einem der vorhergehenden Ansprüche, wobei das Steckergehäuse (30) mit seinen Einzelkomponenten, insbesondere seinen Aufnahmeschalen (30.1,30.2) und dem Außengehäuse (30.9), aus Kunststoff-Spritzgußteilen bestehen und das Kunststoff-Material bevorzugt biokompatibel, sterilisierbar, insbesondere Ethylenoxid-sterilisierbar, ist.

15. Beleuchtungssystem (1) nach mindestens einem der vorhergehenden Ansprüche, wobei das Abstrahlelement (50) ein Zylinder-Diffusor mit im Wesentlichen radialer und/ oder sektional gerichteter Abstrahlcharakteristik, ein sphärischer Diffusor mit im Wesentlichen kugelförmigen Abstrahlverhalten oder ein Frontal-Diffusor mit im Wesentlichen homogener Abstrahlcharakteristik in distaler Richtung ist.

16. Beleuchtungssystem (1) nach mindesten einem der vorhergehenden Ansprüche, wobei bei einer Schwankungsbandbreite der numerischen Apertur NA_{L} der Laser-Lichtquelle (10) des in den Stecker (20) eingekoppelten Laser-Lichtes der Laser-Lichtquelle (10) im Bereich von zumindest 0,08 bis 0,24, bevorzugt auch im Bereich von 0,05 bis 0,30, das Abstrahlverhalten des Abstrahlelementes (50), welches als Zylinder-Diffusor ausgebildet ist, in der Art schwankt, dass die Abstrahlintensität als relative Leuchtdichte (105) gemessen an der Diffusor-Oberfläche über seine Länge nicht mehr als 40 % bezogen auf den Maximal-Wert der relativen Leuchtdichte (105), besonders bevorzugt nicht mehr als 20% abfällt.

## Claims

1. An illumination system (1), comprising at least one laser light source (10) having a numerical aperture NA_{L}, and an optical waveguide (40) which has a connector (20) with a connector housing (30) at a proximal end thereof and which is connectable by said connector (20) to the at least one laser light source (10) and/or can be associated therewith, and which comprises an emitting element (50) at the distal end of the optical waveguide (30), wherein the connector housing (30) has means for reducing an influence of a variation range of the numerical aperture NA_{L} of the laser light source (10) of the laser light from the laser light source (10) coupled into the connector (20).

2. The illumination system (1) according to claim 1, wherein the connector housing (30) comprises at least one guide element (30.4) inside an accommodation portion (30.3) for the optical waveguide (40), for bending the optical waveguide (40) at least partially or in sections thereof.

3. The illumination system (1) according to any one of claims 1 or 2, wherein the at least one guide element (30.4) is in the form of a cylindrical and/or conical pin and/or in the form of a ball, wherein at least inside the accommodation portion (30.3) the optical waveguide (40) is guided in a defined manner in terms of its spatial position such that at least a minimum bending radius (40.1) is maintained.

4. The illumination system (1) according to at least one of claims 1 to 3, wherein the connector housing (30) consists of at least two holding shells (30.1, 30.2) arranged inside the connector housing (30), and wherein at least one of the holding shells (30.1, 30.2) of the housing (30) includes the accommodation portion (30.3) for receiving the optical waveguide (40), wherein the optical waveguide (40) follows an S-shaped or wave-like course, at least section-wise, in the area of the accommodation portion (30.3).

5. The illumination system (1) according to at least one of claims 1 to 4, wherein the area of the accommodation portion (30.3) is in the form of an optical waveguide receiving groove (30.6).

6. The illumination system (1) according to claim 5, wherein the optical waveguide receiving groove (30.6) in at least one of the holding shells (30.1, 30.2) has a depth of at least 1.1 times, preferably at least 2 times the total diameter of the optical waveguide (40) and is preferably U-shaped.

7. The illumination system (1) according to any one of claims 5 or 6, wherein directly adjoining the optical waveguide receiving groove (30.6), at least sections of the wall of the optical waveguide receiving groove (30.6) merge without any step into one or more fixing pins (30.5) which, in the assembled state of the connector housing (30), engage in seats corresponding to the contour of the fixing pins (30.5) in the respective other one of the holding shells (30.1, 30.2).

8. The illumination system (1) according to at least one of the preceding claims, comprising at least one of the following features:
- the connector housing (30) can be assembled from the individual components by means of snap-in connections;
- the connector housing (30) has an inner portion including the accommodation portion (30.3) for the optical waveguide (40), the holding shells (30.1, 30.2), and/or the guide elements (30.4), and an outer housing (30.9), wherein the inner portion is designed to be freely rotatable about a rotation angle of more than 360° relative to the outer housing (30.9);
- the connector housing (30) comprises at least one RFID chip (60) for identifying and/or storing characteristic physical properties of the emitting element (40), and the laser light source (10) has corresponding receiving or reading units;
- the connector housing (30) has means which causes the optical waveguide (40) to be at least partially damaged or cut off or offset after a single use on the laser light source (10) when disconnecting the connector (20) from the laser light source (10) or when disengaging the connection between the laser light source (10) and the connector (20);
- the connector housing (30) comprises a heat sink, in particular in the accommodation portion (30.3).

9. The illumination system (1) according to at least one of the preceding claims, wherein the optical waveguide (40) is in the form of a multimode quartz fibre with a circular, hexagonal or other polygonal or irregular cross-sectional structure of the core.

10. The illumination system (1) according to claim 9, wherein, within the area of the accommodation portion (30.3), a minimum bending radius (40.1) of the optical waveguide (40) in the form of a multimode quartz fibre corresponds to at least 60 times, preferably at least 100 times the diameter of the optical waveguide (40) based on its cladding diameter.

11. The illumination system (1) according to claim 10, wherein, within the area of the accommodation portion (30.3), at the end of the accommodation portion (30.3) downstream of the fibre bend in the direction of light propagation, the multimode quartz fibre that is used as the optical waveguide (40) has at least one portion in which the cladding of the quartz fibre is replaced by a coating material that has a refractive index which is higher than the refractive index of the core of the quartz fibre.

12. The illumination system (1) according to at least one of the preceding claims, wherein the connector (20) is in the form of an SMA or FC connector, and wherein the connector housing (30) has a corresponding connector accommodation portion (30.7) for receiving the connector (20) in a twist-proof manner, wherein a union nut (20.1) for fixing the connector (20) in the laser light source (10) can be fastened in a twist-proof manner in the outer housing (30.9).

13. The illumination system (1) according to at least one of the preceding claims, wherein the accommodation portion (30.3) comprises a mechanical protection element (70) for the optical waveguide (40), in particular a strain relief and/or a kink protection.

14. The illumination system (1) according to at least one of the preceding claims, wherein the connector housing (30) with its individual components, in particular its holding shells (30.1, 30.2) and the outer housing (30.9), consists of injection moulded parts of plastics material, and wherein the plastics material is preferably biocompatible, sterilisable, in particular sterilisable by ethylene oxide.

15. The illumination system (1) according to at least one of the preceding claims, wherein the emitting element (50) is a cylindrical diffuser with a substantially radial and/or sectionally directed emission characteristic, a spherical diffuser with a substantially spherical emission behaviour, or a frontal diffuser with a substantially homogeneous emission characteristic in the distal direction.

16. The illumination system (1) according to at least one of the preceding claims, wherein with a variation range of the numerical aperture NA_{L} of the laser light source (10) within the range of at least 0.08 to 0.24, preferably also within the range from 0.05 to 0.30, of the laser light of the laser light source (10) coupled into the connector (20), the emission behaviour of the emitting element (50) which is in the form of a cylinder diffuser varies such that the emission intensity measured as relative luminance (105) on the surface of the diffuser does not drop by more than 40 % over the length thereof, most preferably by not more than 20 %, based on the maximum value of relative luminance (105).

## Revendications

1. Système d'éclairage (1), comprenant au moins une source de lumière laser (10), dotée d'une ouverture numérique NA_{L}, et un conducteur de lumière (40) qui présente un connecteur (20), doté d'un boîtier de connecteur (30) à une extrémité proximale, et qui peut être raccordé à la source de lumière laser (10), au nombre d'au moins une, au moyen du connecteur (20) et/ou peut être associé à celle-ci, et le système présentant un élément rayonnant (50) à l'extrémité distale du conducteur de lumière (30), le boîtier de connecteur (30) présentant des dispositifs de réduction d'une influence d'une bande de fluctuation de l'ouverture numérique NA_{L} de la source de lumière laser (10) de la lumière laser, couplée dans le connecteur (20), de la source de lumière laser (10).

2. Système d'éclairage (1) selon la revendication 1, dans lequel le boîtier de connecteur (30), dans une partie de réception (30.3) accueillant le conducteur de lumière (40), présente au moins un élément de guidage (30.4) destiné à la flexion, au moins partielle ou par segments, du conducteur de lumière (40).

3. Système d'éclairage (1) selon l'une des revendications 1 ou 2, dans lequel l'élément de guidage (30.4), au nombre d'au moins un, est réalisé sous forme de tige cylindrique et/ou en forme de cône et/ou de sphère, le conducteur de lumière (40) étant guidé, au moins dans la partie de réception (30.3), de manière définie dans l'espace en ce qui concerne sa position, en respectant au moins un rayon de flexion (40.1) minimal.

4. Système d'éclairage (1) selon au moins une des revendications 1 à 3, dans lequel le boîtier de connecteur (30) se compose d'au moins deux coques de réception (30.1, 30.2), disposées à l'intérieur du boîtier de connecteur (30), et au moins une des coques de réception (30.1, 30.2) du boîtier (30) comporte la partie de réception (30.3) destinée à accueillir le conducteur de lumière (40), le conducteur de lumière (40) présentant, au moins par segments, une forme de S ou d'onde, dans la région de la partie de réception (30.3).

5. Système d'éclairage (1) selon l'une des revendications 1 à 4, dans lequel la région de la partie de réception (30.3) est réalisée comme rainure de réception de guide de lumière (30.6).

6. Système d'éclairage (1) selon la revendication 5, dans lequel la rainure de réception de guide de lumière (30.6), dans au moins une des coques de réception (30.1, 30.2), présente une profondeur d'au moins 1,1 fois, de préférence d'au moins 2 fois le diamètre total du conducteur de lumière (40) et est de préférence réalisée en forme de U.

7. Système d'éclairage (1) selon l'une des revendications 5 ou 6, dans lequel, directement sur la rainure de réception de guide de lumière (30.6), la paroi de la rainure de réception de guide de lumière (30.6) se raccordent au moins par segments, sans gradin, à une ou plusieurs tiges de maintien (30.5) qui, à l'état assemblé du boîtier de connecteur (30), s'engagent dans des logements correspondant au contour des tiges de maintien (30.5), respectivement dans l'autre coque de réception (30.1, 30.2).

8. Système d'éclairage (1) selon au moins une des revendications précédentes, présentant au moins une des caractéristiques suivantes :
- le boîtier de connecteur (30) peut être assemblé à partir des éléments individuels au moyen de liaisons par encliquetage,
- le boîtier de connecteur (30) présente une partie intérieure, comportant la partie de réception (30.3) pour le conducteur de lumière (40), les coques de réception (30.1, 30.2) et/ou les éléments de guidage (30.4), ainsi qu'un boîtier extérieur (30.9), la partie intérieure étant réalisée de manière à pouvoir tourner librement avec un angle de rotation de plus de 360° par rapport au boîtier extérieur (30.9),
- le boîtier de connecteur (30) présente au moins une puce RFID (60) pour l'identification et/ou l'enregistrement de propriétés physiques caractéristiques de l'élément rayonnant (40), et la source de lumière laser (10) présente des unités de réception ou de lecture correspondantes appropriées,
- le boîtier de connecteur (30) présente un dispositif avec lequel le conducteur de lumière (40), après une utilisation unique sur la source de lumière laser (10), est endommagé ou coupé ou déplacé au moins en partie lors du retrait du connecteur (20) de la source de lumière laser (10), respectivement lors de la séparation de la liaison entre la source de lumière laser (10) et le connecteur (20),
- le boîtier de connecteur (30) présente un puits thermique, en particulier dans la partie de réception (30.3).

9. Système d'éclairage (1) selon au moins une des revendications précédentes, dans lequel le conducteur de lumière (40) est réalisé sous forme de fibre de quartz multimode à structure de section transversale du coeur circulaire, hexagonale ou ayant une autre forme polygonale ou réalisée avec une forme irrégulière.

10. Système d'éclairage (1) selon la revendication 9, dans lequel un rayon de flexion (40.1) minimal du conducteur de lumière (40) réalisé sous forme de fibre de quartz multimode, dans la région de la partie de réception (30.3), correspond au moins à 60 fois, de préférence au moins à 100 fois le diamètre du conducteur de lumière (40), rapporté à son diamètre d'enveloppe.

11. Système d'éclairage (1) selon la revendication 10, dans lequel la fibre de quartz multimode utilisée comme conducteur de lumière (40) présente, dans la région de la partie de réception (30.3), dans la direction de la propagation de la lumière, à l'extrémité de la partie de réception (30.3), après la flexion de la fibre, au moins une zone partielle où l'enveloppe de la fibre de quartz est remplacée par un matériau de revêtement qui présente un indice de réfraction supérieur à l'indice de réfraction du coeur de la fibre de quartz.

12. Système d'éclairage (1) selon au moins une des revendications précédentes, dans lequel le connecteur (20) est réalisé sous forme de connecteur SMA ou FC, et le boîtier de connecteur (30) présente une partie de réception de connecteur (30.3) correspondante destinée à accueillir le connecteur (20) avec blocage en rotation, un écrou-raccord (20.1), destiné à maintenir le connecteur (20) dans la source de lumière laser (10), pouvant être fixé dans le boîtier extérieur (30.9) en étant bloqué en rotation.

13. Système d'éclairage (1) selon au moins une des revendications précédentes, dans lequel la partie de réception (30.3) présente un élément de protection (70) mécanique pour le conducteur de lumière (40), en particulier un élément antitraction et/ou un élément antiflam-bage.

14. Système d'éclairage (1) selon au moins une des revendications précédentes, dans lequel le boîtier de connecteur (30), avec ses composants individuels, notamment ses coques de réception (30.1, 30.2) et le boîtier extérieur (30.9), est constitué de pièces moulées par injection en matière plastique, et le matériau plastique est de préférence biocompatible, stérilisable, en particulier stérilisable à l'oxyde d'éthylène.

15. Système d'éclairage (1) selon au moins une des revendications précédentes, dans lequel l'élément rayonnant (50) est un diffuseur cylindrique à caractéristique de rayonnement sensiblement radiale et/ou sectionnelle, un diffuseur sphérique à comportement de rayonnement sensiblement sphérique, ou un diffuseur frontal doté d'une caractéristique de rayonnement sensiblement homogène dans la direction distale.

16. Système d'éclairage (1) selon au moins une des revendications précédentes, dans lequel, pour une bande de fluctuation de l'ouverture numérique NA_{L} de la source de lumière laser (10) de la lumière laser, couplée dans le connecteur (20), de la source de lumière laser (10), qui est comprise dans la plage allant d'au moins 0,08 à 0,24, de préférence aussi dans la plage allant de 0,05 à 0,30, le comportement de rayonnement de l'élément rayonnant (50), qui est réalisé comme diffuseur cylindrique, varie de manière à ce que l'intensité de rayonnement, mesurée en tant que densité lumineuse (105) relative sur la surface du diffuseur, ne diminue pas, sur sa longueur, de plus de 40 % par rapport à la valeur maximale de la densité lumineuse (105) relative, et de manière particulièrement avantageuse ne diminue pas de plus de 20 %.
